# EUROPEAN PATENT SPECIFICATION

(11) **EP 2 980 293 B1**
(45) Date of publication and mention of the grant of the patent: **13.12.2017**
(21) Application number: 14775255.4
(22) Date of filing: 26.03.2014
(51) Int. Cl.: D04H 1/542, A61F 13/511, A61F 13/51

(54) **NONWOVEN FABRIC AND PRODUCT OBTAINED USING SAME**
VLIESSTOFF UND DARAUS GEWONNENES PRODUKT
TEXTILE NON-TISSÉ ET PRODUIT OBTENU AU MOYEN DE CELUI-CI

(30) Priority: 28.03.2013 JP 2013070156
(43) Date of publication of application: 03.02.2016
(73) Proprietor: JNC Corporation, Chiyoda-ku Tokyo 100-8105 (JP); JNC Fibers Corporation, Tokyo 100-0004 (JP)
(72) Inventor: TERADA, Hirokazu, Moriyama-shi Shiga 524-0001 (JP)
(74) Representative: Müller-Boré & Partner Patentanwälte PartG mbB
(86) International application number: PCT/JP2014/058424
(87) International publication number: WO 2014/157280

(56) References cited:
- EP-A1- 2 384 881
- EP-A2- 0 171 807
- WO-A1-00/78883
- WO-A1-89/10989
- JP-A- 2001 003 253
- JP-A- 2011 219 873
- JP-A- 2011 219 900
- US-A1- 2011 240 210

## Description

### Technical Field

The invention relates to a nonwoven fabric that can be preferably used for a surface material of a hygienic material or the like.

### Background Art

In general, a nonwoven fabric prepared by performing thermobonding of a web formed of thermobondable fibers in a circulation-type hot-air heat treatment apparatus is formed into a bulky nonwoven fabric (EP 2384881 A1, US 2011/240210 A1). However, in the thus obtained nonwoven fabric, thermobonding is performed at intersections of the fibers and the fibers are fixed wholly over the nonwoven fabric. Therefore, the nonwoven fabric has had a disadvantage of resulting in reduced flexibility of the nonwoven fabric, and further reduced permeability of a highly viscous liquid or the like.

Consequently, development has been made on a nonwoven fabric having high flexibility while maintaining bulkiness. Specifically, a proposal has been made on a nonwoven fabric mainly containing thermo-fusible conjugate fibers composed of a low-melting point component and a high-melting point component, in which the nonwoven fabric is formed of thermally connected region (I) and thermally non-connected region (II), thermobonding is performed by the thermo-fusible conjugate fibers in the thermally connected region (I), and thermobonding is performed at intersections of the fibers without causing compression flattening in the fibers in a thermobonded part, and thermally non-connected region (II) is a part in which no thermobonding is performed (for example, see Patent literature No. 1).

However, while the nonwoven fabric described above has flexibility, thermally non-connected regions (II) continuously exist on a surface of the nonwoven fabric, for example when thermally connected regions (I) are formed into a staggered array, and therefore strength of the nonwoven fabric tends to be reduced. Moreover, the nonwoven fabric described above has a structure in which thermally connected regions (I) giving hard texture are scattered about on thermally non-connected regions (II) giving soft texture. Therefore, the hard texture is further strongly exhibited and tends to roughen the surface of the nonwoven fabric to reduce drape. Further, in thermally non-connected region (II), when the nonwoven fabric becomes wet with water or the like, the fibers in thermally non-connected region (II) are flexed and sunk in the bulkiness. As a result, a liquid once absorbed therein is easily released (easily causes wetback), and absorption performance tends to be reduced.

### Citation List

### Patent Literature

Patent literature No. 1: JP 2001-3253 A.

### Summary of Invention

### Technical Problem

The invention is contemplated for providing a nonwoven fabric having high strength of the nonwoven fabric even with excellent flexibility. The nonwoven fabric to be obtained is expected to be excellent in permeability or absorbency of a highly viscous liquid, and further have an effect of reducing wetback of the liquid.

### Solution to Problem

The present inventor has diligently continued to conduct study in order to solve the problem. As a result, the present inventor has found that a nonwoven fabric prepared by constituting non-thermobonded regions and thermobonded regions having a specific structure in specific arrangement on a surface of the nonwoven fabric can solve the problem, and thus have completed the invention based on the finding.

The invention has structure as described below. A nonwoven fabric, obtained using thermobondable fibers, in which non-thermobonded regions where no thermobonding is performed at intersections of the fibers, and thermobonded regions where thermobonding is performed at the intersections of the fibers alternately exist on a surface of the nonwoven fabric, wherein the non-thermobonded regions exist in a dispersed manner in a dot form, or continuously or intermittently exist in a linear form, and thermobonding is performed at intersections of the fibers without causing compression flattening in the fibers in the thermobonded regions, wherein a thickness of the non-thermobonded region is smaller than a thickness of the thermobonded region in a thickness direction of the nonwoven fabric.

### Advantageous Effects of Invention

A nonwoven fabric of the invention has high strength of the nonwoven fabric even with excellent flexibility. Then, according to a special structure thereof, the nonwoven fabric is excellent in permeability or absorbency of a highly viscous liquid such as menstrual blood and loose stool, and has an effect of reducing wetback of the liquid.

### Brief Description of Drawings

Figure 1 shows one aspect of masking on a masking conveyor in which a plurality of round mask portions (black portions in the drawing) are provided on a mesh conveyor. However, no mesh structure of the mesh conveyor is shown.
Figure 2 shows one aspect of masking on a masking conveyor in which a plurality of linear mask portions (black portions in the drawing) are provided on a mesh conveyor. However, no mesh structure of the mesh conveyor is shown.
Figure 3 shows one aspect of a punching plate in which a plurality of round holes are provided on a metal plate (black in the drawing).

### Description of Embodiments

A nonwoven fabric of the invention is obtained using thermobondable fibers, in which non-thermobonded regions where no thermobonding is performed at intersections of the fibers, and thermobonded regions where thermobonding is performed at the intersections of the fibers alternately exist on a surface of the nonwoven fabric, and the non-thermobonded regions exist in a dispersed manner in a dot form, or continuously or intermittently exist in a linear form, and thermobonding is performed at intersections of the fibers without causing compression flattening in the fibers in the thermobonded regions.

A method for producing the nonwoven fabric according to the invention is not particularly limited, if thermobonding is performed at the intersections of the fibers without causing compression flattening in the fibers in the thermobonded regions. Specific examples of the method for producing the nonwoven fabric include a method in which, in a step of preparing a web using thermobondable fibers, and then allowing thermobonding of the fibers with each other at the intersections of the fibers, the web is placed on a transportation conveyor, and further from above, the web is interposed with a conveyor (hereinafter, referred to as "masking conveyor") formed by partially applying a plurality of masking to the mesh conveyor having sufficient air permeability or the like so as to allow no penetration of hot air thereinto, and the hot air is penetrated from a side of the masking conveyor toward a side of the transportation conveyor by using a circulation-type hot-air heat treatment apparatus, for example. Thermobonding is performed in the thus obtained nonwoven fabric at the intersections of the fibers in parts through which the hot air is penetrated, and no bonding is performed at the intersections of the fibers in parts through which no hot air is penetrated by masking.

In the invention, "thermobonded region" means a region in which bonding is performed at the intersections of the fibers, and particularly means a region through which the hot air is penetrated to perform bonding at the intersections of the fibers. "Non-thermobonded region" means a region in which no thermobonding is performed at the intersections of the fibers, and particularly means a region through which no hot air is penetrated by masking to cause no thermobonding at the intersections of the fibers. Here, a non-thermobonded portion having a pattern corresponding to a mesh shape of the mesh conveyor is formed in the thermobonded region in several cases. However, in the invention, the thermobonded region including the pattern of the non-thermobonded portion generated in the thermobonded region in association with such a production method is also referred to as "thermobonded region."

In the invention, "masking" means masking of other places than the places in which thermobonding is allowed in order to protect such other places during thermobonding. Specifically, such an apparatus can be preferably used for masking as the mesh conveyor having a shielding part (mask) for allowing no penetration of the hot air, and a part without the shielding part for allowing penetration of the hot air.

In the invention, "without causing compression flattening in the fibers in the thermobonded regions" means that no compression flattening is caused, toward a thickness direction of the nonwoven fabric, in the fibers at the intersections at which thermobonding is performed in the fibers of the nonwoven fabric with each other. "Compression flattening" includes a state in which the fibers are formed into a film by thermocompression.

In the invention, "web" means an aggregate of the fibers in a state in which the fibers are entangled in no small way, and the aggregate of the fibers obtained by a carding method, a wet method, a spunbond method or a meltblown method, for example.

In the invention, in order to form the non-thermobonded regions in the nonwoven fabric, the masking conveyor formed by partially applying a plurality of masks to the mesh conveyer so as to allow no penetration of the hot air thereinto only needs to be used as described above. Moreover, in order to allow the non-thermobonded regions to exist in the dispersed manner in the dot form in the nonwoven fabric, individual masks only need to be provided so as to be arrayed in the dispersed manner in the dot form. Moreover, in order to allow the non-thermobonded regions to continuously or intermittently exist in the linear form in the nonwoven fabric, the individual masks only need to be provided so as to be arrayed continuously or intermittently in the linear form.

In the masking, the masks "being arrayed continuously or intermittently in the linear form" means an aspect in which the masks are continuously or intermittently extended in the linear form or a curve form with predetermined periodicity or regularity. Therefore, the non-thermobonded regions "continuously or intermittently existing in the linear form" means the non-thermobonded regions obtained by the masking as described above. In the invention, a width of a line of the mask is not particularly limited. The masks preferably have an aspect in which the masks are continuously extended in the linear form or the curve form in corresponding to MD (abbreviation of a machine direction) or CD (abbreviation of a cross machine direction) of the nonwoven fabric. Moreover, the width of each line of the masks is preferably 0.5 to 30 millimeters, and further preferably 1 to 20 millimeters in view of drape of the nonwoven fabric to be obtained. Moreover, an interval between individual lines of the masks is preferably 0.5 to 20 millimeters, and further preferably 1 to 10 millimeters in view of strength.

Moreover, in the masking, the masks "in the dispersed manner in the dot form" means an aspect in which the masks exist in the dot form, and existence thereof may be irregular (discrete) or regular. Therefore, the non-thermobonded regions "existing in the dispersed manner in the dot form" means the non-thermobonded regions to be obtained by the masking described above. Individual shapes of the masks for masking are not particularly limited, and only need to be a desired shape according to an intended purpose, such as a round shape, an elliptic shape, a quadrangle and a rhombus, and individual sizes are not particularly limited, either. The masks may have each independently a different shape or a different size.

The individual masks for masking are not particularly limited, as long as the non-thermobonded regions are arranged so as to prevent the regions from existing in a linked manner with each other on the surface of the nonwoven fabric. However, the regions are optionally arranged regularly or irregularly (randomly) according to the intended purpose in view of improving the strength or the drape of the nonwoven fabric. With regard to individual "dot forms," when conversion is made into a circle having an area identical with an area of the dot form based on the individual areas, a diameter thereof is preferably 0.5 to 30 millimeters, and further preferably 1 to 10 millimeters in view of the drape. Moreover, an interval between individual "dot forms" is preferably 1 to 20 millimeters, and further preferably 5 to 10 millimeters in view of the strength of the nonwoven fabric. As the mesh conveyor on which the masks are provided, the mesh conveyor having a mesh size of a degree at which the hot air is effectively penetrated thereinto only needs to be adopted, and selected from meshes having the mesh size onto which the mask having the size described above can be attached.

On one side of the nonwoven fabric, a ratio of a total area of the thermobonded regions to a surface area thereof is preferably 40 to 95%, further preferably 60 to 90%, and particularly preferably 60 to 70%. If the ratio of the thermobonded regions is 40% or more, the strength of the nonwoven fabric becomes sufficient. Furthermore, interstices among the fibers are fixed (ensured) by a three-dimensional network structure constituted by mutually linking the thermobonded regions to be formed by thermobonding, and bulkiness of the nonwoven fabric can be maintained. Thus, wetback of the liquid is expected to become difficult. On the other hand, if the ratio of the thermobonded regions is 95% or less, the ratio of the non-thermobonded regions becomes 5% or more, and therefore a predetermined or more amount of the non-thermobonded regions in the nonwoven fabric is secured. The fibers in the non-thermobonded regions exist without being connected with each other. Therefore, when a highly viscous fluid such as loose stool rushes to the non-thermobonded regions at a fixed flow speed, the fibers existing with a degree of freedom in the non-thermobonded regions can be extended in the interstices or changed in shapes of the interstices so as to be pushed with a pressure of the fluid and to be ensured in flow channels of the fluid. In the nonwoven fabric of the invention, favorable permeability of a particularly highly viscous fluid is also expected.

A method for measuring the ratio of the area is as described below. The surface of the nonwoven fabric is observed to measure the area of the thermobonded regions in which thermobonding is performed in constitutional fibers partially in a concentrated manner, and the ratio of the area to the total area of a sample for measurement is calculated. As the sample for measurement, a sample cut into 100 millimeters × 100 millimeters is used (the total area of the sample for measurement is adjusted to 10,000 mm².). Front and back surfaces of the sample for measurement are observed, and areas of the thermobonded regions are measured in ten places for each by using "3D Digital Fine Scope VC2400-IMU Ver.2.3" made by Omron Corporation. The ratio can be determined by calculating mean values (%) on the front and back surfaces.

The nonwoven fabric of the invention can be preferably obtained by applying heat treatment using the hot air while the web is interposed between the conveyer for conveying the web, and the masking conveyor in a heat treatment step. On the above occasion, a clearance of the conveyor therefor and the masking conveyor should be varied depending on the thickness of the web to be used. The clearance between the conveyors only needs to be appropriately adjusted so as to prevent a desired height of the bulkiness from being adversely affected for the nonwoven fabric to be obtained and so as to prevent formation of weak bonding in the non-thermobonded regions due to infiltration of plenty of the hot air into the interstices among the web and parts covered with the masks, and more specifically, so as to sufficiently develop a function of the masking. On the above occasion, the masks may be provided on a surface of the masking conveyor and on a side toward the web, or on a surface on a side opposite to the side of the web. However, the mask is preferably provided on the surface toward the side of the web in order to improve an effect of shielding the hot air with the mask because a masking effect is further improved due to direct contact of a mask portion with the web. Moreover, a structure may be formed in which a thickness of a part of the masking conveyor, the part being covered with the mask, is particularly increased toward the side of the web in order to further improve the masking effect. Thus, infiltration of the hot air into the void between the web and the part covered with the mask can be further effectively suppressed. A thickness of the mask on the above occasion only needs to be appropriately set up in a suitable range within the range in which an original purpose is achieved, and simultaneously the hot air efficiently reaches the web, and within the range in which no industrial disadvantage is caused.

Specific examples of a material of the masking conveyor include a PET net and a metal net. Moreover, the masking conveyor is industrially preferably endless and preferably rotates at a speed identical with a speed of the conveying conveyor. A part of masking is composed by being plugged with a silicone resin or the like.

Specific examples of the thermobondable fibers to be used in the invention include fibers composed of polyolefin, polyester, polyamide, polylactic acid, and various kinds of elastomer resins. The fibers may be mixed and used. Moreover, when the thermobondable fibers are conjugate fibers composed of resins different from each other, specific examples include conjugate fibers formed of a combination of a low-melting point component and a high-melting point component. Specific examples of the combination of the low-melting point component and the high-melting point component include polyethylene-polypropylene, polypropylene copolymer-polypropylene homopolymer and polyethylene-polyethylene terephthalate, but the combination is not limited thereto. In the invention, as the polyethylene, a polyethylene homopolymer, a copolymer containing ethylene as a main component, the copolymer of ethylene and propylene or any other olefin, and a copolymer containing ethylene as a main component, the copolymer of ethylene and any other copolymerizable component, can be used. Moreover, as the polypropylene, a polypropylene homopolymer, a copolymer containing propylene as a main component, the copolymer of propylene and ethylene or any other olefin, and a copolymer containing propylene as a main component, the copolymer of propylene and any other component can be used. As the polyester, polyethylene terephthalate, polybutylene terephthalate and a copolymer thereof can be used.

In the conjugate fibers, a melting point of the high-melting point component is preferably 10°C or more higher than a melting point of the low-melting point component. When the conjugate fibers are constituted of the resins different from each other, in particular, two kinds of resins (the low-melting point component and the high-melting point component) having different melting points from each other, an area ratio (low-melting point component / high-melting point component) in a cross-section of the fiber of the conjugate fibers in a direction perpendicular to a length direction is preferably 10/90 to 90/10, and further preferably 40/60 to 60/40.

Conditions of the heat treatment using the hot air only need to be appropriately set up according to kinds of resins to be used and constituting the thermobondable fibers. For example, as a temperature of the hot air, the heat treatment only needs to be applied at the temperature at which the surface of the thermobondable fibers is thermally melted to cause thermobonding at the intersections of the thermobondable fibers with each other. Moreover, an air speed or the like of the hot air is not particularly limited, and can be appropriately adopted from the conditions of the heat treatment to be ordinarily applied using the circulation-type hot-air heat treatment apparatus or the like.

In the web, other fibers may be mixed in the thermobondable fibers if other fibers are within the range in which advantageous effects of the invention are not significantly adversely affected. Specific examples of other fibers include natural fibers such as wood fibers, regenerated fibers such as rayon, semi-synthetic fibers such as acetate fibers and synthetic fibers such as the fibers of acryl, nylon and polyvinyl chloride. If such other fibers are fibers engaging with no thermobonding, the number of points of bonding among the fibers in the nonwoven fabric to be obtained from the web can be reduced, which can further provide the thus obtained nonwoven fabric with flexibility.

A fineness of the fibers such as the thermobondable fibers and other fibers to be used in the invention is preferably 1.0 to 11 dtex, and further preferably 1.5 to 5.5 dtex. Moreover, as the fibers, continuous fibers (long fibers) or short fibers can be used. However, short fibers having a fiber length of 10 to 120 millimeters are preferred, and short fibers having a fiber length of 30 to 60 millimeters are further preferred. If the flexibility of the nonwoven fabric is taken into consideration, the fibers having comparatively low fineness should be selected. Moreover, when the fibers are the conjugate fibers, specific examples of the cross-section thereof include a concentric sheath-core type, an eccentric sheath-core type and a side-by-side type, but the cross-section is not particularly limited if the cross-section is within the range in which the advantageous effects of the invention are not significantly adversely affected.

In the nonwoven fabric of the invention, no compression flattening is caused in the fibers constituting the thermobonded regions, and thermobonding is performed at the intersections among the fibers. A production method for obtaining such a constitution is not particularly limited, but the constitution can be preferably obtained by a method such as hot-air bonding without depending on thermocompression or the like by using a heated embossing roll.

The nonwoven fabric to be obtained according to the invention can be favorably used for allowing permeation of a comparatively highly viscous liquid such as menstrual blood and loose stool. In contrast, the nonwoven fabric thermobonded, for example in the circulation-type hot-air heat treatment apparatus is formed into a bulky nonwoven fabric, but thermobonding is performed in the nonwoven fabric wholly at the intersections of the fibers with each other, and the fibers are three-dimensionally fixed. Therefore, no fibers therein are allowed to freely move. In such a nonwoven fabric, in order to allow permeation of the highly viscous liquid in a thickness direction, the liquid is to pass among the fibers that are three-dimensionally stretched, and the thermobondable fibers block flow of the liquid. Therefore, the particularly highly viscous liquid becomes hard to perform the liquid permeation.

Moreover, for example, with regard to the liquid permeation through the nonwoven fabric obtained by performing thermocompression of the web using a heated roll such as an embossing roll, a thickness of the nonwoven fabric becomes small as a whole. Therefore, while a liquid permeation time is shortened in comparison with the nonwoven fabric prepared by thermobonding in the circulation-type hot-air heat treatment apparatus as described above, a portion bonded by thermocompression allows no liquid permeation because compression flattening is caused in the fibers into a film form. Moreover, also in a region other than the portion bonded by thermocompression, the thermobondable fibers are wholly heated in a process of passing through the heated roll to form a bonded state in some degree. Therefore, no fibers are allowed to freely move, and in a similar manner as described above, such a region serves as a factor of blocking the liquid permeation. Furthermore, the web is pressed with adjacent projections of the embossing roll, and the nonwoven fabric in a position corresponding to recesses interposed between both projections is pushed down on both sides. As a result, the thickness thereof is also reduced. Therefore, the thickness of the nonwoven fabric obtained is generally small, and as the whole, the nonwoven fabric lacks in water retention properties, and the liquid once absorbed therein easily gets back (large in wetback).

On the other hand, the nonwoven fabric of the invention has, in a mixed manner, the thermobonded regions where thermobonding is performed at the intersections of the fibers with each other, and the non-thermobonded regions where no thermobonding is performed in the fibers with each other, and the fibers can freely move only by entanglement of the fibers with each other. Therefore, part of the highly viscous liquid passes through the fabric while avoiding the thermobondable fibers that are three-dimensionally stretched, and the liquid comes to partially pass through the non-thermobonded regions, namely the regions in which the thermobondable fibers can freely move. In the non-thermobonded regions, the thermobondable fibers can freely move, and therefore no regions block the liquid permeation. Thus, such regions are excellent in permeation of the particularly highly viscous liquid. Further, the liquid passes through the non-thermobonded regions. Thus, the thermobondable fibers become wet with the liquid, and flexed (sunk), and the bulkiness of the nonwoven fabric in the thickness direction is reduced. Therefore, the liquid is facilitated to flow in a further lower layer. The above matters also have a role of inhibiting backflow of the liquid once permeated as well as a capability of shortening the liquid permeation time. The reason is that sites serving as the thermobonded regions maintain the bulkiness by bonding of the fibers in the wetback of the liquid to block the wetback with the bulkiness (hollow walls). Thus, absorption performance can be adjusted according to a field in which the fabric is used by adjusting the ranges of the thermobonded regions and the non-thermobonded regions within the range described herein.

In the nonwoven fabric of the invention, in view of the points described above, the thickness of the non-thermobonded region is preferably smaller than the thickness of the thermobonded region in the thickness direction of the nonwoven fabric. When the nonwoven fabric of the invention is produced by placing the web on the transportation conveyor, and further from above, interposing the web with the masking conveyor, and blowing the hot air thereonto from the side of the masking conveyor, with regard to the web in the regions covered with the mask portion of the masking conveyor, no connection is made in fibers constituting the web with each other, and no three-dimensional network structure is fixed among the fibers. Therefore, the fibers are compressed in the thickness direction of the web in receiving an air pressure of the hot air to be applied to a mask member and a weight of the masking conveyor itself. Then, even after the above loads are eliminated, the fibers are still compressed, and insufficiently restored to original bulkiness because no three-dimensional network structure is originally fixed in a relationship among the fibers. On the other hand, while non-masking portions directly receive the hot air, the fibers are three-dimensionally fixed by thermobonding in the relationship among the fibers. Therefore, after the air pressure is eliminated, force trying to restore the bulkiness acts on the web pushed by the hot air. The thickness of the non-thermobonded region is considered to become smaller than the thickness of the thermobonded region due to phenomena described above. In such a nonwoven fabric, a difference in the thickness can be preferably controlled by appropriately setting a processing temperature, a circulation air speed and a clearance between the conveyors.

The nonwoven fabric of the invention may have a monolayer or a plurality of layers. When the plurality of layers are formed, layers other than the nonwoven fabric according to the invention may be laminated thereon and used. Specific examples of other layer include an air-laid layer and a spunbond layer.

A product may be obtained using the nonwoven fabric prepared using the thermobondable fibers, in which the non-thermobonded regions where no thermobonding is performed at the intersections of the fibers, and the thermobonded regions where thermobonding is performed at the intersections of the fibers alternately exist on the surface of the nonwoven fabric, and the non-thermobonded regions exist in the dispersed manner in the dot form, or continuously exist in the linear form, and thermobonding is performed at the intersections thereof without causing compression flattening in the fibers in the thermobonded regions.

Specific examples of the product obtained using the nonwoven fabric according to the invention include a disposable diaper, a sanitary item and a product as a hygienic material such as an absorbent sheet. Further, the product can be preferably used for a disposable wiper or the like.

### Examples

The invention will be described in greater detail by way of Examples below, but the invention is not limited to the Examples.

### Fibers

Thermobondable fibers described below were used in Examples and Comparative Examples.

Fiber A: concentric sheath-core conjugate fibers having a fineness of 2.2 dtex and a cut length of 51 mm, and having a zigzag crimp, in which polyester (SP1912SD, IV value: 0.63, made by Chung Shing Textile Co., Ltd.) was used as a core component, and HDPE (high density polyethylene, S6900, MI: 16, made by Keiyo Polyethylene Co., Ltd.) was used as a sheath component.

Fiber B: eccentric sheath-core conjugate fibers having a fineness of 3.3 dtex and a cut length of 51 mm, and having a helical crimp, in which polypropylene (SA2E, MFR: 15, made by Japan Polypropylene Corporation) was used as a core component, and HDPE (high density polyethylene, S6900, MI: 16, made by Keiyo Polyethylene Co., Ltd.) was used as a sheath component.

### Preparation of web

A web having a basis weight of 25 g/m² was prepared using the fibers described above as a raw material and using a miniature carding machine, made by Daiwa-kiko Corporation Ltd.

### Nonwoven fabric processing

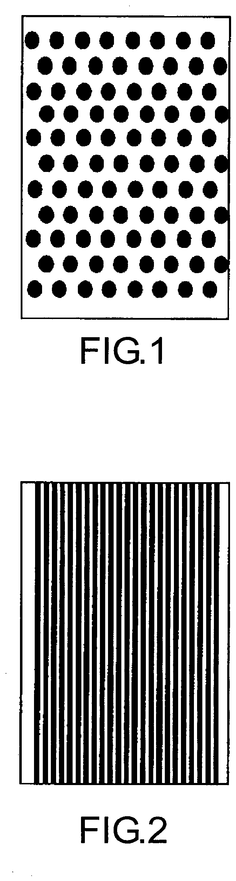

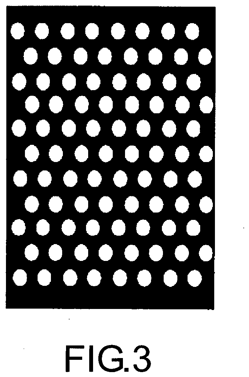

Under processing conditions of a hot air temperature of 130°C, an air speed of 0.5 m/s and a conveyor speed of 8.5 m/min in a hot-air circulation-type heat treatment apparatus, a masking conveyor was placed on the web prepared by the method described above, and then heat treatment was applied thereto. A masking conveyor a or b described below was used therefor. Moreover, punching plate c was placed thereon under the identical conditions, and the heat treatment was applied thereto:
a: masking conveyor in an aspect in Figure 1 in which a diameter of one mask in a circular form was 5 mm and a thickness thereof was 2 mm, and the masks were arrayed at a pitch of 5 mm in a staggered form;
b: masking conveyor in an aspect in Figure 2 in which a width of one mask in a linear form was 2 mm and a thickness was 2 mm, and the masks were arrayed at a pitch of 1 mm so as to be extended in an MD direction; and
c: punching plate in an aspect in Figure 3 in which a diameter of one hole in a circular form was 3 mm, and the holes were arrayed at a pitch of 5 mm in a staggered form.

### Observation of cross-section of nonwoven fabric

A nonwoven fabric was perpendicularly cut from a surface so as to pass through both thermobonded regions and non-thermobonded regions, and a cross-section of the nonwoven fabric was observed by a microscope (VHX-600) made by KEYENCE Corporation.

### Evaluation of flexibility of nonwoven fabric

Flexibility was measured in accordance with JIS L1096, bending resistance-A method (45°C cantilever method) except that a width of a sample was adjusted to 50 mm (20 mm in JIS). As a numerical value of measured bending resistance was smaller, the sample was evaluated to be a nonwoven fabric having higher drape properties and flexibility. As the evaluation, a sample being significantly flexible in both directions of MD and CD or significantly flexible in any one of the directions was taken as "very good," a sample being flexible was taken as "good" and a sample being poor in the flexibility due to many bonded points was taken as "poor."

### Evaluation of strength of nonwoven fabric

With regard to strength of a nonwoven fabric, ten monitors perform tension and tearing of samples each having a width of 50 mm and a sample length of 150 mm, and a sample judged to have significant strength was taken as "very good," a sample judged to have strength was taken as "good" and a sample judged to have only a limited number of bonded points and no strength was taken as "poor."

### Example 1

A web having a basis weight of 25 g/m² was used in which fiber A was used and the web was obtained by a method of preparation of web described above. Masking conveyor a was placed on the web such that a side of a surface subjected to masks was placed on a side of the web, and a nonwoven fabric was produced in a hot-air circulation heat treatment apparatus. No thermobonding was caused in parts subj ected to masking (also referred to as parts covered with the masks) on the nonwoven fabric obtained, and non-thermobonded regions were formed. Thermobonding was caused in fibers with each other at intersections of the fibers in parts other than the above, into which hot air was penetrated, and thermobonded regions were formed. A thickness of the non-thermobonded region was confirmed to be smaller than a thickness of the thermobonded region (0.7 mm and 1.8 mm, respectively) by observation of cross-section of the nonwoven fabric. The nonwoven fabric was significantly flexible in both directions of MD and CD because the non-thermobonded regions were uniformly scattered wholly about the nonwoven fabric.

### Example 2

A web having a basis weight of 25 g/m² was used in which fiber B was used and the web was obtained by a method of preparation of web described above. Masking conveyor b was placed on the web such that a side of a surface subjected to masks was placed on a side of the web, and a nonwoven fabric was obtained in a hot-air circulation heat treatment apparatus. No thermobonding was caused in parts subjected to masking on the nonwoven fabric obtained, and non-thermobonded regions were formed. Thermobonding was caused in fibers with each other at intersections of the fibers with each other in parts other than the above, into which hot air was penetrated, and thermobonded regions were formed. A thickness of the non-thermobonded region was confirmed to be smaller than a thickness of the thermobonded region (0.7 mm and 1.4 mm, respectively) by observation of cross-section of the nonwoven fabric. The nonwoven fabric was significantly flexible particularly in CD because the non-thermobonded regions went straight ahead toward MD in the nonwoven fabric.

### Comparative Example 1

A web having a basis weight of 25 g/m² was used in which fiber A was used and the web was obtained by a method of preparation of web described above. A nonwoven fabric was produced in a hot-air circulation heat treatment apparatus without using any masking conveyor on the web. Hot air was penetrated wholly into the web, and thus thermobonding was caused in the nonwoven fabric obtained at intersections of the fibers with each other, and a thickness of the nonwoven fabric was uniform for all (1.3 mm). The nonwoven fabric was poor in flexibility because the nonwoven fabric was composed only of thermobonded regions.

### Comparative Example 2

A web having a basis weight of 25 g/m² was used in which fiber B was used and the web was obtained by a method of preparation of web described above. A nonwoven fabric was produced in a hot-air circulation heat treatment apparatus without using any masking conveyor on the web. Hot air was penetrated wholly into the web, and thus thermobonding was caused in the nonwoven fabric obtained at intersections of the fibers with each other, and a thickness of the nonwoven fabric was uniform for all. The nonwoven fabric became significantly high in bulkiness and poor in flexibility because the nonwoven fabric was composed only of thermobonded regions and a helical crimp was generated in fiber B by hot air.

### Comparative Example 3

A nonwoven fabric was produced by using a web having a basis weight of 25 g/m² in which fiber A was used and the web was obtained by a method of preparation of web described above, and applying heating compression using a flat roll and an embossing roll having an embossing area ratio of 25% and heated to 120°C. While embossed compressed portions were formed into a film by thermocompression of the fibers with each other, and regions between embossed points were in a lightly bonded state by heat from upper and lower rolls, while a fiber shape remained.

### Comparative Example 4

A nonwoven fabric was obtained using a web having a basis weight of 25 g/m² in which fiber A was used and the web was obtained by a method of preparation of web described above, and placing punching plate c on the web in a hot-air circulation heat treatment apparatus. Hot air was penetrated through punching parts on the nonwoven fabric obtained, and thermobonding was caused in fibers with each other at intersections of the fibers with each other, and thermobonded regions were formed. Other parts were formed into non-thermobonded regions, and a thickness thereof was confirmed to be smaller than a thickness of the thermobonded regions (0.7 mm and 1.2 mm, respectively). The nonwoven fabric was significantly flexible particularly in CD, but significantly low in strength because the non-thermobonded regions went straight ahead toward MD.

**Table 1**

| | Fibers | Nonwoven fabric processing | Flexibility | Strength of nonwoven fabric |
|---|---|---|---|---|
| Example 1 | A | Masking conveyor a | Very good | Very good |
| Example 2 | B | Masking conveyor b | Very good | Good |
| Comparative Example 1 | A | No masking conveyor | Poor | Very good |
| Comparative Example 2 | B | No masking conveyor | Poor | Very good |
| Comparative Example 3 | A | Embossing processing | Poor | Good |
| Comparative Example 4 | A | Masking conveyor c | Very good | Poor |

### Industrial Applicability

A nonwoven fabric of the invention maintains favorable strength of the nonwoven fabric while maintaining high flexibility. Then, the nonwoven fabric can be preferably used for a product such as a surface material of a hygienic material because the nonwoven fabric has favorable absorbency from structural features, is excellent in absorbency of a particularly highly viscous liquid, and expected to have difficulty in causing wetback.

## Claims

1. A nonwoven fabric, obtained using thermobondable fibers, in which non-thermobonded regions where no thermobonding is performed at intersections of the fibers, and thermobonded regions where thermobonding is performed at the intersections of the fibers alternately exist on a surface of the nonwoven fabric, wherein the non-thermobonded regions exist in a dispersed manner in a dot form, or continuously or intermittently exist in a linear form, and thermobonding is performed at the intersections of the fibers without causing compression flattening in the fibers in the thermobonded regions, wherein a thickness of the non-thermobonded region is smaller than a thickness of the thermobonded region in a thickness direction of the nonwoven fabric.

## Patentansprüche

1. Vliesstoff, erhalten unter Verwendung von thermobondierbaren Fasern, bei dem nicht-thermobondierte Bereiche, in denen keine Thermobondierung an Kreuzungen der Fasern durchgeführt wird, und thermobondierte Bereiche, in denen das Thermobondieren an den Schnittpunkten der Fasern durchgeführt wird, abwechselnd auf einer Oberfläche des Vliesstoffs vorhanden sind, wobei die nicht-thermobondierten Bereiche in einer dispergierten Weise in einer Punktform vorliegen oder kontinuierlich oder intermittierend in einer linearen Form vorliegen, und das Thermobondieren an den Schnittpunkten der Fasern durchgeführt wird, ohne eine Kompressionsabflachung in den Fasern in den thermobonierten Bereichen zu bewirken, wobei eine Dicke des nicht-thermobondierten Bereichs kleiner als eine Dicke des thermobondierten Bereichs in einer Dickenrichtung des Vliesstoffs ist.

## Revendications

1. Tissu non tissé, obtenu en utilisant des fibres pouvant être thermosoudées, dans lequel des zones non thermosoudées, dans lesquelles aucun thermosoudage n'est exécuté aux intersections des fibres, et des zones thermosoudées, dans lesquelles un thermosoudage est exécuté aux intersections des fibres, sont agencées de manière alternée sur une surface du tissu non tissé, dans lequel les zones non thermosoudées sont agencées d'une manière dispersée sous une forme de point, ou sont agencées de manière continue ou intermittente sous une forme linéaire, et le thermosoudage est exécuté aux intersections des fibres sans provoquer d'aplatissement par compression sur les fibres dans les zones thermosoudées, dans lequel une épaisseur de la zone non thermosoudée est inférieure à une épaisseur de la zone thermosoudée dans le sens de l'épaisseur du tissu non tissé.
